(19) Europäisches Patentamt
European Patent Office
Office européen des brevets

(11) EP 1 550 430 B1

(12) **FASCICULE DE BREVET EUROPEEN**

(45) Date de publication et mention de la délivrance du brevet:
**06.05.2009 Bulletin 2009/19**

(51) Int Cl.:
***A61K 8/891*** *(2006.01)* ***A61Q 1/02*** *(2006.01)*
***A61Q 1/10*** *(2006.01)*

(21) Numéro de dépôt: **04292993.5**

(22) Date de dépôt: **14.12.2004**

(54) **Composition cosmétique solide comprenant des fibres**

Fasern enthaltende feste kosmetische Zusammensetzung

Solid cosmetic composition containing fibers

(84) Etats contractants désignés:
**AT BE BG CH CY CZ DE DK EE ES FI FR GB GR HU IE IS IT LI LT LU MC NL PL PT RO SE SI SK TR**

(30) Priorité: **19.12.2003 FR 0351128**

(43) Date de publication de la demande:
**06.07.2005 Bulletin 2005/27**

(73) Titulaire: **L'ORÉAL**
**75008 Paris (FR)**

(72) Inventeurs:
- **Delacour, Marie-Laure**
**75013 Paris (FR)**
- **Ray, Xavier**
**91580 Villeconin (FR)**

(74) Mandataire: **Boulard, Denis**
**L'OREAL - D.I.P.I.**
**25-29 Quai Aulagnier**
**92600 Asnières (FR)**

(56) Documents cités:
**EP-A- 1 023 893**
**EP-A- 1 025 837**
**EP-A- 1 051 968**
**EP-A- 1 064 930**
**US-A1- 2004 071 648**

EP 1 550 430 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention a pour objet une composition solide, notamment cosmétique, comprenant des particules d'organopolysiloxane élastomère et des fibres. L'invention a également pour objet un procédé de maquillage ou de traitement non thérapeutique des matières kératiniques d'être humain, telles que la peau, les ongles, les cils, les sourcils, les cheveux, et en particulier de la peau, comprenant l'application de la composition sur les matières kératiniques.

La composition selon l'invention peut être une composition de maquillage ou de soin des matières kératiniques, en particulier de la peau, et de préférence une composition de maquillage.

La composition de maquillage peut être un produit de maquillage de la peau, tel qu'un produit de maquillage du teint (notamment fond de teint), un fard à paupières, un eye-liner, un fard à joue, un produit anticernes, un produit de maquillage du corps, un produit de maquillage des lèvres, un produit de maquillage des ongles, un produit de maquillage des cheveux. De préférence, la composition est une composition de maquillage de la peau, en particulier un fond de teint, un fard à paupières, un fard à joues. Plus spécialement, l'invention porte sur une composition de maquillage du teint, notamment un fond de teint.

La composition de soin de la peau peut être un produit de soin de la peau (du visage,du corps, des mains), un produit matifiant de la peau, un produit de protection solaire de la peau (notamment du visage), une composition autobronzante, un produit déodorant.

Il est connu de la demande WO 02/053126 des compositions cosmétiques solides comprenant des particules d'organopolysiloxane réticulé élastomère en milieu aqueux et des poudres telles que des pigments et des charges ; ces compositions peuvent présenter une texture pâteuse cohésive de forme solide. Ces compositions solides sont notamment conditionnées dans une coupelle et présentent une surface de prélèvement ayant un caractère élastique procurant ainsi un toucher original et différent de celui des poudres compactes classiques dont le toucher est dur, rigide.

Pour utiliser les compositions solides décrites dans la demande WO 02/053126, l'utilisatrice frotte la surface du produit solide à l'aide d'un applicateur tel qu'une éponge, une houppette ou un pinceau, ou bien au doigt pour prélever la quantité désiré de produit destinée à être appliquée sur les matières kératiniques à traiter ou à maquiller. Or, du fait de la texture particulière (notamment souple et élastique) du produit, ce dernier se prélève difficilement avec l'applicateur et lorsque l'utilisatrice frotte trop fortement la surface du produit, la surface peut se détériorer en se fragmentant ou en se fissurant : la surface du produit devient alors irrégulière et inesthétique, et la prise du produit est encore plus difficile. Le produit ne présente plus un aspect attrayant pour l'utilisatrice et devient donc rédhibitoire.

Le but de la présente invention est donc de disposer d'une composition solide comprenant des particules d'organopolysiloxane réticulé élastomère et des poudres et pouvant se prélever à l'aide d'un applicateur ou au doigt sans détériorer la surface du produit.

Le demandeur a découvert qu'une telle composition est obtenue en introduisant des fibres dans la composition, les fibres permettant de renforcer la résistance aux cisaillements de la surface du produit et ainsi d'éviter la détérioration de la surface lors de la prise du produit avec un applicateur : au cours de son utilisation, le produit ne présente pas de fragmentation en surface, ni de fissures et conserve un aspect de surface régulier et esthétique.

Il est connu de la demande EP1064930 une composition cosmétique comprenant des particules d'organopolysolixane réticulé élastomère et des fibres pour obtenir un maquillage homogène, uniforme et un toucher doux. Ce document ne préconise pas d'employer les fibres pour renforcer la résistance aux cisaillements d'un produit solide comprenant des poudres.

De façon plus précise, l'invention a pour objet une composition solide comprenant de l'eau, des particules solides d'organopolysiloxane élastomère, des fibres et une poudre additionnelle. La composition est en particulier une composition cosmétique.

L'invention a également pour objet un procédé cosmétique de maquillage ou de traitement non thérapeutique des matières kératiniques, notamment de la peau, comprenant l'application sur les mattières kératiniques, notamment sur la peau, d'une composition telle que définie précédemment.

On entend par composition solide une composition qui ne s'écoule pas sous son propre poids à température ambiante (25 °C) au bout d'une heure.

La composition selon l'invention contient des particules d'organopolysiloxane réticulé élastomère.
L'organopolysiloxane réticulé élastomère peut être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déhydrogénation entre un diorganopolysiloxane à terminaisons hydroxyle et un diorganopolysiloxane contenant au moins un hydrogène lié au silicium, notamment en présence d'un organoétain ; ou par réaction de condenstaion réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation (A) de diorganopolysiloxane contenant au moins deux hydrogènes liés chacun à un silicium, et (B) de diorganopolysiloxane ayant au moins deux groupements à insaturation éthylénique (notamment groupes vinyliques) liés au silicium, notamment en présence (C) de catalyseur platine, comme par exemple décrit dans la demande EP-A-295886.

En particulier, l'organopolysiloxane peut être obtenu par réaction de diméthylpolysiloxane à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins 2 atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique.

Le composé (A) peut avoir une viscosité à 25 °C allant de 1 à 50 000 centistokes, notamment pour être bien miscible avec le composé (B).

Les groupes organiques liés aux atomes de silicium du composé (A) peuvent être des groupes alkyles tels que méthyl, éthyl, propyl, butyl, octyl ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (B) est avantageusement un diorganopolysiloxane ayant au moins deux groupes alkényles inférieur (par exemple en C2-C4) ; le groupe alkényle inférieur peut être choisi parmi les groupes vinyl, allyl, et propényl. Ces groupements alkényles inférieurs peuvent être situés en toute position de la molécule organopolysiloxane mais sont de préférence situés aux extrémité de la molécule organopolysiloxane. L'organopolysiloxane (B) peut avoir une structure chaîne ramifiée, chaîne linéaire, cyclique ou réseau mais la structure chaîne linéaire est préférée. Le composé (B) peut avoir une viscosité allant de l'état liquide à l'état de gomme. De préférence, le composé (B) a une viscosité d'au moins 100 centistokes à 25 °C.

Les organopolysiloxanes (B) peuvent être choisi parmi les méthylvinylsiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

Outre les groupes alkényle précités, les autres groupes organiques liés aux atomes de silicium dans le composé (B) peuvent être des groupes alkyles tels que méthyl, éthyl, propyl, butyl ou octyl ; des groupes alkyles substitués tels que 2-phényléthyl, 2-phénylpropyl ou 3,3,3-trifluoropropyl ; des groupes aryles tels que phényl, tolyl ou xylyl ; des groupes aryles substitués tels que phényléthyl ; et des groupes hydrocarbonés monovalents substitués tels qu'un groupe époxy, un groupe ester carboxylate, ou un groupe mercapto.

Les organopolysiloxanes (B) peuvent être choisis parmi les méthylvinylpolysiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

En particulier, l'organopolysiloxane élastomère peut être obtenu par réaction de diméthylpolysiloxane à terminaisons diméthylvinylsiloxy et de méthylhydrogénopolysiloxane à terminaisons triméthylsiloxy, en présence de catalyseur platine.

Avantageusement, la somme du nombre de groupements éthyléniques par molécule du composé (B) et du nombre d'atomes d'hydrogène liés à des atomes de silicium par molécule du composé (A) est d'au moins 5.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1,5/1 à 20/1.

Le composé (C) est le catalyseur de la réaction de réticulation , et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en

poids, en tant que métal platine propre pour 1000 parts en poids de la quantité totale des composés (A) et (B).

De telles particules d'organopolysiloxane réticulé élastomère sont notamment décrites dans les demandes JP-A-61-194009, EP-A-242219, EP-A-381166.

Avantageusement, l'élastomère est un élastomère non-émulsionnant. Le terme "non émulsionnant" définit des élastomères organopolysiloxane ne contenant pas de motifs polyoxyalkylène.

Comme élastomères sous forme de poudre, on peut utiliser ceux vendus sous les dénominations "DC9505", "DC 9506" par la société Dow Corning,

Selon un mode particulier de réalisation de l'invention, les particules d'organopolysiloxane réticulé élastomère utilisées peuvent se présenter sous la forme d'une dispersion aqueuse.

Les organopolysiloxanes élastomériques selon l'invention peuvent être choisis parmi les polymères réticulés décrits dans la demande JP-A-10/175816 ou le brevet US 5928660. Selon cette demande, ils sont obtenus par réaction d'addition et de réticulation, en présence d'un catalyseur en particulier du type platine, d'au moins :

- (a) un organopolysiloxane (i) ayant au moins deux groupes vinyliques en position $\alpha$-$\omega$ de la chaîne siliconée par molécule ; et
- (b) un organosiloxane (ii) ayant au moins un atome d'hydrogène lié à un atome de silicium par molécule.

En particulier, l'organopolysiloxane (i) est choisi parmi les polydiméthylsiloxanes et est plus spécifiquement un $\alpha$-$\omega$-diméthylvinyl polydiméthylsiloxane.

Les organopolysiloxanes élastomériques de la composition selon l'invention se présente avantageusement sous forme de suspension aqueuse. Cette suspension peut être notamment obtenue comme suit :

- (a) mélange d'un organopolysiloxane (i) ayant au moins deux groupes vinyliques en position $\alpha$-$\omega$ de la chaîne siliconée par molécule et d'un organosiloxane (ii) ayant au moins un atome d'hydrogène lié à un atome de silicium par molécule ;
- (b) ajout d'un catalyseur, en particulier de type platine ;
- (b) ajout d'une phase aqueuse contenant un émulsifiant pour former une émulsion ;
- (c) polymérisation de l'organopolysiloxane (i) et de l'organosiloxane (ii) en émulsion en présence d'un catalyseur de platine.

L'émulsionnant peut être choisis parmi les tensioactifs non ioniques, cationiques ou anioniques de HLB $\geq$ 8, de préférence choisi parmi les tensioactifs non ioniques.

La proportion de tensioactifs est de préférence de 0,1 à 20 parties en poids pour 100 parties en poids de la composition d'organopolysiloxane élastomérique, et mieux, de 0,5 à 10 parties en poids (cf. description du document JP-A-10/175816).

Après l'étape (c), il est possible de sécher les particules obtenues, pour en évaporer toute ou partie de l'eau piégée.

Les organopolysiloxanes peuvent être sous la forme de particules solides déformables ayant une certaine dureté, mesurable avec un duromètre Shore A (selon la norme ASTM D2240) à la température ambiante ou avec la méthode japonaise JIS-A. Cette dureté peut être mesurée sur un bloc d'élastomère préparé à cet effet comme suit : mélange de l'organopolysiloxane (i) et de l'organosiloxane (ii) ; élimination de l'air du mélange ; moulage et vulcanisation au four à 100°C pendant 30 minutes ; refroidissement à température ambiante puis mesure de la dureté. La densité est aussi déterminée sur ce bloc d'élastomère.

L'organopolysiloxane peut avoir une dureté JIS-A inférieure ou égale à 80, notamment allant de 10 à 80), et de préférence inférieure ou égale à 65, notamment allant de 15 à 65.

Comme particules d'organopolysiloxane en dispersion dans l'eau, on peut utiliser celles commercialisées sous les noms BY 29-122 et BY 29-119 par la société Dow-Corning.

Les particules d'organopolysiloxane réticulé élastomère peuvent être présentes dans la composition selon l'invention en une teneur allant de 10 % à 50 % en poids, par rapport au poids total de la composition, de préférence allant de 20 % à 40 % en poids, et préférentiellement allant de 25 % à 40 % en poids.

La composition selon l'invention contient également des fibres.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 1,5 à 2500, de préférence de 5 à 500, et mieux de 3,5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 $\mu$m à 10 mm, de préférence de 0,1 mm à 5 mm et mieux de 0,3 mm à 1 mm . Leur section peut être comprise dans un cercle de diamètre allant de 2 nm à 500 $\mu$m, de préférence allant de 100 nm à 100 $\mu$m et mieux de 1 $\mu$m à 50 $\mu$m. Le poids ou titre des fibres est souvent donné en denier ou décitex et représente le poids en gramme pour 9 km de fil. De préférence, les fibres selon l'invention ont un titre choisi dans la gamme allant de 0,01 à 10 à deniers, de préférence de 0,1 à 2 deniers et mieux de 0,3 à 0,7 deniers.

Les fibres peuvent être celles utilisées dans la fabrication des textiles et notamment des fibres de soie, de coton, de laine, de lin, des fibres de cellulose - notamment extraites notamment du bois, des légumes ou des algues -, de rayonne, de polyamide (Nylon® ), de viscose, d'acétate notamment d'acétate de rayonne, de poly-(p-phénylène-téréphtalamide) (ou d'aramide) notamment de Kevlar® , de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène (comme le Téflon® ), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.
De préférence, les fibres sont des fibres de polyamide (Nylon® ).

On peut aussi utiliser les fibres utilisées en chirurgie comme les fibres synthétiques résorbables préparées à partir d'acide glycolique et caprolactone ("Monocryl" de chez Johnson & Johnson) ; les fibres synthétiques résorbables du type copolymère d'acide lactique et d'acide glycolique ("Vicryl" de chez Johnson & Johnson) ; les fibres de polyester téréphtalique ("Ethibond" de chez Johnson & Johnson) et les fils d'acier inoxydable ("Acier" de chez Johnson & Johnson).

Par ailleurs, les fibres peuvent être traités ou non en surface, enrobées ou non d'une couche de protection.

Comme fibres enrobées utilisables dans l'invention, on peut citer des fibres de polyamides enrobées de sulfure de cuivre pour un effet anti-statique (par exemple le "R-STAT" de chez Rhodia) ou un autre polymère permettant une organisation particulière des fibres (traitement de surface spécifique). On peut encore citer les fibres enrobées par des pigments minéraux ou organiques, tels que les pigments cités plus loin dans la demande.

De préférence, on utilise des fibres d'origine synthétique et en particulier des fibres organiques, comme celles utilisées en chirurgie.

Les fibres utilisables dans la composition selon l'invention sont préférentiellement des fibres de polyamide, de cellulose, de poly-p-phénylène téréphtalamide ou de de polyéthylène. Leur longueur (L) peut aller de 0,1 mm à 5 mm, de préférence de 0,25 mm à 1,6 mm et leur diamètre moyen peut aller de 1 $\mu$m à 50 $\mu$m. En particulier, on peut utiliser les fibres de polyamide commercialisées par les Etablissements P. Bonte sous le nom de "Polyamide 0.9 Dtex", ayant un diamètre moyen de 6 $\mu$m, un titre d'environ 0.9 dtex et une longueur allant de 0,3 mm à 5 mm, ou bien encore les fibres de polyamides vendues sous la dénomination Fiber Ion 931-D1-S par la société LCW , ayant un titre d'environ 0,9 dtex et une longueur d'environ 0,3 mm. On peut aussi utiliser les fibres de celluloses (ou de rayonne) ayant un diamètre moyen de 50 $\mu$m et une longueur allant de 0,5 mm à 6 mm comme celles vendues sous le nom de "Natural rayon flock fiber RC1BE - N003 - M04" par la société Claremont Flock. On peut également utiliser des fibres de polyéthylène comme celles vendues sous le nom de "Shurt Stuff 13 099 F" par la société Mini Fibers.

La composition selon l'invention peut également comprendre des fibres dites "rigides", par opposition aux fibres citées précédemment, qui ne sont pas des fibres rigides.

Les fibres rigides, initialement sensiblement droites, lorsqu'elles sont placées dans un milieu dispersant, ne voient pas leur forme sensiblement modifiée, ce qui se traduit par la condition angulaire définie ci-après, reflétant une forme que l'on peut qualifier de toujours, sensiblement droite, linéaire. Cette condition d'angle reflète la rigidité des fibres qui peut difficilement être exprimée par un autre paramètre pour des objets ayant une taille aussi faible que les fibres rigides.

La rigidité des fibres se traduit par la condition angulaire suivante : avantageusement, au moins 50 % en nombre, de préférence au moins 75 % en nombre, et mieux au moins 90 % en nombre des fibres sont telles que l'angle formé entre la tangente à l'axe central longitudinal de la fibre et la droite reliant ladite extrémité au point sur l'axe central longitudinal de la fibre correspondant à la moitié de la longueur de la fibre soit inférieur à 15 ° et l'angle formé entre la tangente à l'axe central longitudinal de la fibre en un point situé à mi-longueur de la fibre et la droite reliant l'une des extrémités au point sur l'axe central longitudinal de la fibre correspondant à la moitié de la longueur de fibre soit inférieur ou égal à 15 °, pour une même longueur de fibre allant de 0,8 mm à 5 mm, de préférence allant de 1 mm à 4 mm, de préférence allant de 1 mm à 3 mm, et mieux de 2 mm.
Avantageusement, l'angle, mentionné ci-dessus, est mesuré aux deux extrémités de la fibre et en un point situé à mi-longueur de la fibre, en d'autres termes, on effectue dans ce cas trois mesures et la moyenne des angles mesurés est inférieure ou égale à 15°.
Notamment, la tangente, en tout point de la fibre, forme un angle inférieur à 15°.
Dans la présente demande, l'angle formé par la tangente en un point de la fibre est l'angle formé entre la tangente à l'axe central longitudinal de la fibre audit point de la fibre et la droite reliant l'extrémité de la fibre la plus proche dudit point au point sur l'axe central longitudinal de la fibre correspondant à la moitié de la longueur de la fibre.

Généralement, les fibres rigides utilisables dans la composition selon l'invention ont la même longueur de fibre ou une longueur sensiblement identique.

Plus précisément lorsque l'on observe au microscope, avec un objectif permettant un grossissement de 2,5 et avec une vision plein champ, un milieu dans lequel sont dispersées les fibres rigides à une concentration des fibres de 1 % en poids, un nombre majoritaire

de fibres rigides, c'est-à-dire au moins 50 % en nombre des fibres rigides, de préférence au moins 75 % en nombre des fibres rigides, et mieux au moins 90 % en nombre des fibres rigides, doivent satisfaire à la condition angulaire définie plus haut. La mesure conduisant à la valeur de l'angle est effectuée pour une même longueur de fibres, cette longueur est comprise dans la gamme allant de 0,8 mm à 5 mm, de préférence de 1 à 4 mm, de préférence de 1 à 3 mm, et mieux de 2 mm.

Le milieu dans lequel est réalisée l'observation est un milieu dispersant assurant une bonne dispersion des fibres rigides, par exemple de l'eau, un gel aqueux d'argile ou de polyuréthane associatif. On peut même réaliser une observation directe de la composition contenant les fibres rigides. Un échantillon de la composition ou de la dispersion préparée est placée entre lame et lamelle pour l'observation au microscope avec un objectif permettant un grossissement de 2,5 et avec une vision plein champ. La vision plein champ permet de voir les fibres dans leur intégralité.

Les fibres rigides peuvent être choisies parmi les fibres d'un polymère synthétique choisi parmi les polyesters, les polyuréthanes, les polymères acryliques, les polyoléfines, les polyamides, en particulier les polyamides non aromatiques et les polyimides-amides aromatiques.

Comme exemples de fibres rigides, on peut citer les fibres:

- de polyesters, telles que celles obtenues par découpe de fils vendus sous les dénominations FIBRE 255-100-R11-242T TAILLE 3 MM (section octalobée), FIBRE 265-34-R11-56T TAILLE 3 MM (section ronde), FIBRE COOLMAX 50-34-591 TAILLE 3 MM (section tétralobée) par la société DUPONT DE NEMOURS ;

- de polyamide, telles que celles vendues sous les dénominations TRILOBAL NYLON 0.120-1.8 DPF ; TRILOBAL NYLON 0.120-18 DPF ; NYLON 0.120-6 DPF par la société Cellusuede products ; ou obtenues par découpe de fils vendus sous la dénomination FIBRE NOMEX BRAND 430 TAILLE 3 MM par la société DUPONT DE NEMOURS ;

- de polyimide-amide telles que celles vendues sous les dénomination "KERMEL", "KERMEL TECH" par la société RHODIA ;

- de poly-(p-phénylène-téréphtalamide) (ou d'aramide) notamment vendues sous la dénomination Kevlar® par la société DUPONT DE NEMOURS ;

- des fibres à structure multicouche comprenant des couches alternées de polymères choisis parmi les polyesters, les polymères acryliques, les polyamides, tellles que celles décrites dans les documents EP-A-6921217, EP-A-686858 et US-A-5472798. De telles fibres sont vendues sous les dénominations "Morphotex", « Teijin Tetron Morphotex » par la société TEIJIN.

Les fibres rigides particulièrement préférées sont les fibres de polyimide-amide aromatiques.

Des fils ou fibres de polyimide-amide, qui peuvent être utilisés pour les compositions de l'invention, sont décrits, par exemple, dans le document de R. PIGEON et P. ALLARD, Chimie Macromoléculaire Appliquée, 40/41 (1974), pages 139-158 (n° 600), ou bien encore dans les documents US-A-3 802 841, FR-A-2 079 785, EP-A1-0 360 728, EP-A-0 549 494, auxquels on pourra se référer.

Les fibres de polyimide-amide aromatique préférées sont des fibres de polyimide-amide comprenant des motifs répétitif de formule :

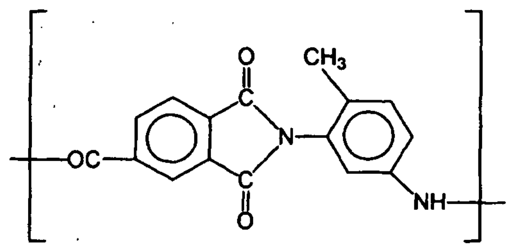

obtenu par polycondensation du toluylène diisocyanate et de l'anhydride triméllitique.

Les fibres peuvent êtres présentes dans la composition selon l'invention en une teneur allant de 0,5 % à 20 % en poids, par rapport au poids total de la composition, de préférence de 0,5 % à 15 % en poids, préférentiellement allant de 0,5 % à 8 % en poids, et plus préférentiellement allant de 2 % à 8 % en poids.

Avantageusement, les fibres et les particules d'organopolysiloxane réticulé élastomère sont présentes dans la composition selon l'invention en des teneurs telles que le rapport pondéral fibres/particules d'organopolysiloxane réticulé va de 0,01 à 2, de préférence va de 0,03 à 1, préférentiellement va de 0,05 à 0,5, et plus préférentiellement va de 0,05 à 0,2.

La composition selon l'invention contient au moins une poudre additionnelle, différente des particules d'organopolysiloxane réticulé élastomère et des fibres décrites précédemment.

La poudre additionnelle peut être choisie parmi les matières colorantes pulvérulentes, les charges et leurs mélanges.

La matière colorante pulvérulente peut être notamment choisie parmi les pigments, les nacres, et leurs mélanges.

Par pigments, il faut comprendre des particules de toute forme, blanches ou colorées, minérales ou organiques, insolubles dans le milieu physiologique, destinées à colorer la composition.

Par nacres, il faut comprendre des particules de toute

forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Les matières colorantes pulvérulentes peuvent être présentes dans la composition selon l'invention en une teneur allant de 0,5 % à 60 % en poids, par rapport au poids total de la composition, de préférence allant de 0,5 % à 50 % en poids, préférentiellement allant de 0,5 % à 40 % en poids, plus préférentiellement allant de 1 % à 30 % en poids, et encore plus préférentiellement allant de 3 % à 25 % en poids.

La composition selon l'invention peut comprendre des charges.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique ( par exemple feuillet, cubique, hexagonale, orthorombique, etc). On peut citer le talc, le mica, la silice, le kaolin, les poudres de polyamide (Nylon® ) , de poly-β-alanine et de polyéthylène, les poudres de polymères de tétrafluoroéthylène (Téflon® ), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les poudres de copolymère diméthacrylate d'éthylèneglycol et de méthacrylate de lauryle notamment vendues sous la dénomination POLYTRAP® 6603 adsorber par la société RP Scherer, les microbilles de résine de silicone (Tospearls® de Toshiba, par exemple), le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, et leurs mélanges.

De préférence, les charges sont choisies parmi le mica, les poudres de polyamide, les poudres de copolymère diméthacrylate d'éthylèneglycol et de méthacrylate de lauryle, le nitrure de bore, et leurs mélanges.

Les charges peuvent être présentes dans la composition en une teneur allant de 0,5 % à 40 % en poids, par rapport au poids total de la composition, de préférence allant de 1 % à 35 % en poids, et préférentiellement allant de 5 % à 30 % en poids.

Avantageusement, la composition selon l'invention peut comprendre une teneur totale en poudres additionnelles (donc matière colorante + charges) allant de 10 % à 70 % en poids, par rapport au poids total de la composition, préférentiellement allant de 10 % à 60 % en poids, et plus préférentiellement allant de 20 % à 50 % en poids.

De préférence, les particules d'organopolysiloxane réticulé élastomère et la poudre additionnelle sont présentes dans la composition selon l'invention en des teneurs telles que le rapport pondéral particules d'organopolysiloxane réticulé/poudre additionnelle va de 0,4/1 à 2,5/1, de préférence va de 0,4/1 à 2/1, préférentiellement va de 0,4/1 à 1,5/1, plus préférentiellement va de 0,4/1 à 1,3/1, et encore plus préférentiellement va de 0,6/1 à 1,3/1.

La composition selon l'invention comprend de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale.

La composition peut comprendre de l'eau en une teneur allant de 5 % à 30 % en poids, par rapport au poids total de la composition, de préférence allant de 10 % à 25 % en poids, préférentiellement allant de 15 % à 25 % en poids, et plus préférentiellement allant de 20 % à 25 % en poids.

La composition peut comprendre également un polyol ayant notamment de 2 à 20 atomes de carbones, de préférence ayant de 2 à 10 atomes de carbone, et préférentiellement ayant de 2 à 6 atomes de carbones. Comme polyol, on peut citer la glycérine, le propylène glycol, le butylène glycol, le pentylène glycol, l'hexylène glycol, le dipropylène glycol, le diéthylène glycol, et leurs mélanges.

La composition selon l'invention peut comprendre un polyol en une teneur allant de 1 % à 20 % en poids, par rapport au poids total de la composition, et de préférence allant de 3 % à 15 % en poids.

La composition peut contenir d'autres ingrédients cosmétiques usuels pouvant être choisis notamment parmi les antioxydants, les parfums, les conservateurs, les neutralisants, les tensioactifs, les cires, les huiles, les filtres solaires, les vitamines, les hydratants, les

composés auto-bronzants, les actifs antirides.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Avantageusement, la composition selon l'invention peut avoir une dureté allant de 0,07 N à 0,4 N, de préférence allant de 0,1 à 0,35 N et une élasticité EL allant de 15 % à 80 %, et de préférence allant de 30 % à 70 %.

La dureté et l'élasticité du produit sont mesurés à 20 °C à l'aide du texturomètre vendu sous la dénomination TA-TX2i par la société RHEO, équipé d'un mobile en inox en forme de bille de diamètre 12,7 mm en mesurant l'évolution de la force (force de compression ou force d'étirement) (F) en fonction du temps, pendant l'opération suivante :

Le mobile est déplacé à la vitesse de 0,1 mm /s puis pénètre dans le produit jusqu'à une profondeur de pénétration de 0,3 mm. Lorsque le mobile a pénétré dans le produit à la profondeur de 0,3 mm, le mobile est retiré à la vitesse de 0,1 mm/s. Pendant le retrait du mobile, la force (force de compression) décroît fortement jusqu'à devenir nulle au bout d'un temps t . Pendant l'opération, le mobile effectue un aller retour en 6 secondes.

La dureté correspond à la force maximale de compression mesurée pendant l'opération ; elle est exprimée en Newton.

L'élasticité EL exprimée en pourcentage est déterminée par la relation:

$$EL\ (\%) = 100 \times (t-3)/(6-3)$$

La composition selon l'invention peut être préparée en mélangeant les divers ingrédients, notamment soit dans un mélangeur/granulateur à turbine de type Baker-Perkins, soit dans un malaxeur bi-vis continue type extrudeur-malaxeur BC21 de la société CLEXTRAL.

La composition peut être conditionnée dans une coupelle ou un boîtier par pressage du mélange des ingrédients.

Avantageusement, la composition selon l'invention ne contient pas le mélange suivant (les pourcentages étant exprimés en poids du poids total de la composition) :

8,1 % de dioxyde de titane
1,9 % d'oxydes de fer
10 % de poudre de nylon
5 % de fibres de polyamide
5 % de poudre acrylique
37,1 % de particules organopolysiloxane élastomère dispersées dans 21,8 % d'eau
5 % de glycérine
5 % de propylène glycol
1 % de conservateur,

L'invention est illustrée plus en détails par les exemples décrits ci-après.

**<u>Exemple 1</u> :**

On a préparé un produit de maquillage du teint ayant la composition suivante:

- Dispersion aqueuse de particules de polydiméthylsiloxane réticulé à 63 % en poids de polymère réticulé
  (BY 29-119 de Dow Corning) 59 g soit 37,1g MA
- Glycérine 5 g
- Propylène glycol 5 g
- Conservateurs 1 g
- Poudre de nylon (Orgasol® 2002 extra D NAT COS d'ATOFINA) 10 g
- Poudre de copolymère diméthacrylate d'éthylèneglycol et de méthacrylate de lauryle vendue sous la dénomination POLYTRAP® 6603 adsorber par la société RP Scherer 5 g
- Fibres de polyamide 0,9 Dtex de longueur 0,3 mm de la société Paul Bonte 5 g
- Pigments (oxydes de fer, dioxyde de titane) 10 g

Après mélange des ingrédients, le produit est conditionné dans une coupelle par pressage. Le produit est prélevé en surface à l'aide d'une éponge, sans détériorer la surface du produit.

Le produit obtenu a une dureté de 0,2 N et une élasticité de 55 % mesurées selon les conditions décrites précédemment.

**<u>Exemple 2</u> :**

On a préparé un produit matifiant pour la peau ayant la composition suivante:

- Dispersion aqueuse de particules de polydiméthylsiloxane
  réticulé à 63 % en poids de polymère réticulé
  (BY 29-119 de Dow Corning) 59 g soit 37,1 g MA
- Glycérine 5 g
- Propylène glycol 5 g
- Conservateurs 1 g
- Poudre de nylon (Orgasol® 2002 extra D NAT COS d'ATOFINA) 20 g
- Poudre de copolymère diméthacrylate d'éthylèneglycol et de méthacrylate de lauryle vendue sous la dénomination POLYTRAP® 6603 adsorber par la société RP Scherer 5 g
- Fibres de polyamide 0,9 Dtex de longueur 0,3 mm

de la société Paul Bonte 5 g

Après mélange des ingrédients, le produit est conditionné dans une coupelle par pressage. Le produit est prélevé en surface à l'aide d'une éponge, sans détériorer la surface du produit.

**Exemple 3** :

On a préparé un fard à paupières ayant la composition suivante:

- Particules de polydiméthylsiloxane réticulé réticulé (DC9506 de Dow Corning) 28 g
- Eau 17 g
- Glycérine 5 g
- Conservateurs 1 g
- Fibres de polyamide 0,9 Dtex de longueur 0,3 mm de la société Paul Bonte 4 g
- Nacres 45 g

Après mélange des ingrédients, le produit est conditionné dans une coupelle par pressage. Le produit est prélevé en surface à l'aide d'une éponge, sans détériorer la surface du produit.

**Exemple 4 :**

On a préparé un produit de maquillage du teint ayant la composition suivante:

- Dispersion aqueuse de particules de polydiméthylsiloxane
  réticulé à 63 % en poids de polymère réticulé
  (BY 29-119 de Dow Corning) 55 g soit 34,65 g MA
- Glycérine 10 g
- Conservateurs 1 g
- Poudre de nylon (Orgasol® 2002 extra D NAT COS d'ATOFINA) 19 g
- Fibres de polyamide 0,9 Dtex de longueur 0,3 mm de la société Paul Bonte 5 g
- Pigments (oxydes de fer, dioxyde de titane) 10 g

Après mélange des ingrédients, le produit est conditionné dans une coupelle par pressage. Le produit est prélevé en surface à l'aide d'une éponge, sans détériorer la surface du produit.

**Exemple 5** :

On a préparé un produit matifiant pour la peau ayant la composition suivante :

- Dispersion aqueuse de particules de polydiméthylsiloxane
  réticulé à 63 % en poids de polymère réticulé
  (BY 29-119 de Dow Corning) 55 g soit 34,65 g MA
- Glycérine 10 g
- Conservateurs 1 g
- Poudre de nylon (Orgasol® 2002 extra D NAT COS d'ATOFINA) 29 g
- Fibres de polyamide 0,9 Dtex de longueur 0,3 mm de la société Paul Bonte 5 g

Après mélange des ingrédients, le produit est conditionné dans une coupelle par pressage. Le produit est prélevé en surface à l'aide d'une éponge, sans détériorer la surface du produit.

**Exemple 6 :**

On a préparé un produit de maquillage du teint ayant la composition suivante:

- Particules de polydiméthylsiloxane réticulé réticulé (DC9506 de Dow Corning) 40g
- Eau 19g
- Glycérine 5 g
- Propylène glycol 5 g
- Conservateurs 1 g
- Poudre de nylon (Orgasol® 2002 extra D NAT COS d'ATOFINA) 10 g
- Poudre de copolymère diméthacrylate d'éthylèneglycol et de méthacrylate de lauryle vendue sous la dénomination POLYTRAP® 6603 adsorber par la société RP Scherer 5 g
- Fibres de polyamide 0,9 Dtex de longueur 0,3 mm de la société Paul Bonte 5 g
- Pigments (oxydes de fer, dioxyde de titane) 10 g

Après mélange des ingrédients, le produit est conditionné dans une coupelle par pressage. Le produit est prélevé en surface à l'aide d'une éponge, sans détériorer la surface du produit.

**Exemple 7 :**

On a préparé un produit matifiant pour la peau ayant la composition suivante:

- Particules de polydiméthylsiloxane réticulé réticulé (DC9506 de Dow Corning) 40g
- Eau 19g
- Glycérine 5 g
- Propylène glycol 5 g
- Conservateurs 1 g
- Poudre de nylon (Orgasol® 2002 extra D NAT COS d'ATOFINA) 20 g
- Poudre de copolymère diméthacrylate d'éthylèneglycol et de méthacrylate de lauryle vendue sous la dénomination - POLYTRAP® 6603 adsorber par la société RP Scherer 5 g

- Fibres de polyamide 0,9 Dtex de longueur 0,3 mm de la société Paul Bonte 5 g

Après mélange des ingrédients, le produit est conditionné dans une coupelle par pressage. Le produit est prélevé en surface à l'aide d'une éponge, sans détériorer la surface du produit.

## Revendications

1. Composition solide comprenant de l'eau, des particules solides d'organopolysiloxane élastomère, des fibres et une poudre additionnelle.

2. Composition selon la revendication précédente, **caractérisée par le fait que** l'organopolysiloxane réticulé est choisi parmi ceux obtenus :

   - par réaction d'addition réticulation de diorganosiloxane contenant au moins un hydrogène lié au silicium et de diorganopolysiloxane ayant des groupements à insaturation éthylénique liés au silicium ;
   - par réaction de condensation réticulation déhydrogénation entre un diorganopolysiloxane à terminaisons hydroxyle et un diorganopolysiloxane contenant au moins un hydrogène lié au silicium ;
   - par réaction de condensation réticulation d'un diorganopolysiloxane à terminaisons hydroxyle et d'un organopolysilane hydrolysable ;
   - par réticulation thermique d'organopolysiloxane ;
   - par réticulation d'organopolysiloxane par radiations de haute énergie.

3. Composition selon la revendication 1 ou 2, **caractérisée par le fait que** l'organopolysiloxane réticulé est obtenu par réaction d'addition réticulation (A) de diorganopolysiloxane contenant au moins deux hydrogènes liés chacun à un silicium, et (B) de diorganopolysiloxane ayant au moins deux groupements à insaturation éthylénique liés au silicium, notamment en présence (C) de catalyseur platine.

4. Composition selon l'une quelconque des revendications 1 à 3, **caractérisée par le fait que** l'organopolysiloxane réticulé est obtenu par réaction de diméthylpolysiloxane à terminaisons diméthylvinylsiloxy et de méthylhydrogénopolysiloxane à terminaisons triméthylsiloxy, en présence de catalyseur platine.

5. Composition selon l'une quelconque des revendications précédentes, **caractérisée par le fait que** les particules d'organopolysiloxane réticulé élastomère sont présentes en une teneur allant de 10 % à 50 % en poids, par rapport au poids total de la composition, de préférence allant de 20 % à 40 % en poids, et préférentiellement allant de 25 % à 40 % en poids.

6. Composition selon l'une quelconque des revendications précédentes, **caractérisée par le fait que** les fibres sont choisies parmi les fibres de soie, de coton, de laine, de lin, des fibres de cellulose, de polyamide, de viscose, d'acétate notamment d'acétate de rayonne, de poly-p-(phénylène-téréphtalamide), en acrylique notamment de polyméthacrylate de méthyle ou de poly 2-hydroxyéthyl méthacrylate, de polyoléfine et notamment de polyéthylène ou de polypropylène, de silice, de carbone notamment sous forme graphite, de polytétrafluoroéthylène, de collagène insoluble, de polyesters, de polychlorure de vinyle ou de vinylidène, d'alcool polyvinylique, de polyacrylonitrile, de chitosane, de polyuréthane, de polyéthylène phtalate, des fibres de mélanges de polymères, les fibres rigides sensiblement rectilignes et leurs mélanges.

7. Composition selon l'une quelconque des revendications précédentes, **caractérisée par le fait que** les fibres sont des fibres d'origine synthétique.

8. Composition selon l'une quelconque des revendications précédentes, **caractérisée par le fait que** les fibres ont une longueur allant de 1 $\mu$m à 10 mm, de préférence de 0,1 mm à 5 mm et mieux de 0,3 à 1 mm.

9. Composition selon l'une quelconque des revendications précédentes, **caractérisée par le fait que** les fibres ont un section comprise dans un cercle de diamètre allant de 2 nm à 500 $\mu$m, de préférence allant de 100 nm à 100 $\mu$m.

10. Composition selon l'une quelconque des revendications précédentes, **caractérisée par le fait que** les fibres ont une longueur L et un diamètre D tel que UD est choisi dans la gamme allant de 1,5 à 2 500, de préférence de 3,5 à 500 et mieux de 5 à 150.

11. Composition selon l'une quelconque des revendications précédentes, **caractérisée** le fait que les fibres sont présentes en une teneur allant de 0,5 % à 20 % en poids, par rapport au poids total de la composition, de préférence de 0,5 % à 15 % en poids, préférentiellement allant de 0,5 % à 8 % en poids, et plus préférentiellement allant de 2 % à 8 % en poids.

12. Composition selon l'une quelconque des revendications précédentes, **caractérisée par le fait que** les fibres et les particules d'organopolysiloxane réticulé élastomère sont présentes en des teneurs telles que le rapport pondéral fibres/particules d'organopolysiloxane réticulé va de 0,01 à 2, de préférence va de

0,03 à 1, préférentiellement va de 0,05 à 0,5, et plus préférentiellement va de 0,05 à 0,2.

**13.** Composition selon l'une quelconque des revendications précédentes, **caractérisée par le fait que** la poudre additionnelle est choisie parmi les matières colorantes pulvérulentes, les charges et leurs mélanges.

**14.** Composition selon l'une quelconque des revendications précédentes, **caractérisée par le fait que** la poudre additionnelle comprend une matière colorante pulvérulente.

**15.** Composition selon la revendication 13 ou 14, **caractérisée par le fait que** la matière colorante pulvérulente est choisie parmi les pigments, les nacres et leurs mélanges.

**16.** Composition selon l'une quelconque des revendications 13 à 15, **caractérisée par le fait que** la matière colorante pulvérulente est choisie parmi le dioxyde de titane, les oxydes de zirconium, les oxydes de cérium, les oxydes de zinc, les oxydes de fer, les oxydes de chrome, le violet de manganèse, le bleu outremer, l'hydrate de chrome, le bleu ferrique, la poudre d'aluminium, la poudre de cuivre.

**17.** Composition selon l'une quelconque des revendications 13 à 16, **caractérisée par le fait que** la matière colorante pulvérulente est présente en une teneur allant de 0,5 % à 60 % en poids, par rapport au poids total de la composition, de préférence allant de 0,5 % à 50 % en poids, préférentiellement allant de 0,5 % à 40 % en poids, plus préférentiellement allant de 1 % à 30 % en poids, et encore plus préférentiellement allant de 3 % à 25 % en poids.

**18.** Composition selon l'une quelconque des revendications précédentes, **caractérisée par le fait que** la poudre additionnelle comprend une charge.

**19.** Composition selon la revendication précédente, **caractérisée par le fait que** la charge est choisie parmi le talc, le mica, la silice, le kaolin, les poudres de polyamide, les poudres de poly-β-alanine, les poudres de polyéthylène, les poudres de polymères de tétrafluoroéthylène, la lauroyl-lysine, l'amidon, le nitrure de bore, les microsphères creuses de chlorure de polyvinylidène/acrylonitrile, les microsphères creuses de copolymères d'acide acrylique, les poudres de copolymère diméthacrylate d'éthylèneglycol et de méthacrylate de lauryle, les microbilles de résine de silicone, le carbonate de calcium précipité, le carbonate de magnésium, l'hydrocarbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, et leurs mélanges.

**20.** Composition selon la revendication 18 ou 19, **caractérisée par le fait que** la charge est choisie parmi le mica, les poudres de polyamide, les poudres de copolymère diméthacrylate d'éthylèneglycol et de méthacrylate de lauryle, le nitrure de bore, et leurs mélanges.

**21.** Composition selon l'une quelconque des revendications 18 à 20, **caractérisée par le fait que** la charge est présente en une teneur allant de 0,5 % à 40 % en poids, par rapport au poids total de la composition, de préférence allant de 1 % à 35 % en poids, et préférentiellement allant de 5 % à 30 % en poids.

**22.** Composition selon l'une quelconque des revendications précédentes, **caractérisée par le fait qu'**elle comprend une teneur totale en poudre additionnelle allant de 10 % à 70 % en poids, par rapport au poids total de la composition, préférentiellement allant de 10 % à 60 % en poids, et plus préférentiellement allant de 20 % à 50 % en poids.

**23.** Composition selon l'une quelconque des revendications précédentes, **caractérisée par le fait que** les particules d'organopolysiloxane réticulé élastomère et la poudre additionnelle sont présentes en des teneurs telles que le rapport pondéral particules d'organopolysiloxane réticulé/poudre additionnelle va de 0,4/1 à 2/1, préférentiellement va de 0,4/1 à 1,5/1, plus préférentiellement va de 0,4/1 à 1,3/1, et encore plus préférentiellement va de 0,6/1 à 1,3/1.

**24.** Composition selon l'une quelconque des revendications précédentes, **caractérisée par le fait que** l'eau est présente en une teneur allant de 5 % à 30 % en poids, par rapport au poids total de la composition, de préférence allant de 10 % à 25 % en poids, préférentiellement allant de 15 % à 25 % en poids, et plus préférentiellement allant de 20 % à 25 % en poids.

**25.** Composition selon l'une quelconque des revendications précédentes, **caractérisée par le fait qu'**elle comprendre un polyol ayant notamment de 2 à 20 atomes de carbone, de préférence ayant de 2 à 10 atomes de carbone, et préférentiellement ayant de 2 à 6 atomes de carbone.

**26.** Composition selon la revendication précédente, **caractérisée par le fait que** le polyol est choisi parmi la glycérine, le propylène glycol, le butylène glycol, le pentylène glycol, l'hexylène glycol, le dipropylène glycol, le diéthylène glycol, et leurs mélanges.

**27.** Composition selon la revendication 25 ou 26, **carac-**

**térisée par le fait que** le polyol est présent en une teneur allant de 1 % à 20 % en poids, par rapport au poids total de la composition, et de préférence allant de 3 % à 15 % en poids.

**28.** Composition selon l'une quelconque des revendications précédentes, **caractérisée par le fait qu'**elle comprend un ingrédient cosmétique choisi parmi les antioxydants, les parfums, les conservateurs, les neutralisants, les tensioactifs, les cires, les huiles, les filtres solaires, les vitamines, les hydratants, les composés auto-bronzants, les actifs antirides.

**29.** Composition selon l'une quelconque des revendications précédentes, **caractérisée par le fait que** la composition a une dureté allant de 0,07 N à 0,4 N, de préférence allant de 0,1 à 0,35 N et a une élasticité EL allant de 15 % à 80 %, et de préférence allant de 30 % à 70 %.

**30.** Composition selon l'une quelconque des revendications précédentes, **caractérisée par le fait que** la composition est une composition cosmétique.

**31.** Composition selon l'une quelconque des revendications précédentes, **caractérisée par le fait que** la composition est une composition de maquillage ou de soin des matières kératiniques, en particulier de la peau.

**32.** Composition selon l'une quelconque des revendications précédentes, **caractérisée par le fait que** la composition est un produit de maquillage du teint (notamment fond de teint), un fard à paupières, un fard à joue, un produit anticernes, un produit de maquillage du corps, un produit de maquillage des lèvres, un produit de maquillage des ongles, un produit de maquillage des ongles, un produit de maquillage des cheveux.

**33.** Composition selon l'une quelconque des revendications précédentes, **caractérisée par le fait que** la composition est une composition de maquillage de la peau, en particulier une composition de maquillage du teint, notamment un fond de teint.

**34.** Composition selon l'une quelconque des revendications 1 à 31, **caractérisée par le fait que** la composition de soin de la peau est un produit de soin de la peau, un produit matifiant de la peau, un produit de protection solaire de la peau, une composition autobronzante, un produit déodorant.

**35.** Procédé de maquillage des matières kératiniques, notamment de la peau, comprenant l'application sur les matières kératiniques, notamment sur la peau, d'une composition selon l'une quelconque des revendications précédentes.

**Claims**

**1.** Solid composition comprising water, solid particles of elastomeric organopolysiloxane, fibres and an additional powder.

**2.** Composition according to the preceding claim, **characterized in that** the crosslinked organopolysiloxane is chosen from those obtained:

- via a crosslinking addition reaction of diorganosiloxane containing at least one hydrogen linked to silicon and of diorganopolysiloxane containing ethylenically unsaturated groups linked to silicon;
- via a dehydrogenation crosslinking condensation reaction between a diorganopolysiloxane containing hydroxyl end groups and a diorganopolysiloxane containing at least one hydrogen linked to silicon;
- via a crosslinking condensation reaction of a diorganopolysiloxane containing hydroxyl end groups and of a hydrolysable organopolysilane;
- via thermal crosslinking of organopolysiloxane;
- via crosslinking of organopolysiloxane by high-energy radiation.

**3.** Composition according to Claim 1 or 2, **characterized in that** the crosslinked organopolysiloxane is obtained via a crosslinking addition reaction (A) of diorganopolysiloxane containing at least two hydrogens each linked to a silicon, and (B) of diorganopolysiloxane containing at least two ethylenically unsaturated groups linked to silicon, especially in the presence (C) of a platinum catalyst.

**4.** Composition according to any one of Claims 1 to 3, **characterized in that** the crosslinked organopolysiloxane is obtained via reaction of dimethylpolysiloxane containing dimethylvinylsiloxy end groups and of methylhydrogenopolysiloxane containing trimethylsiloxy end groups, in the presence of a platinum catalyst.

**5.** Composition according to any one of the preceding claims, **characterized in that** the particles of elastomeric crosslinked organopolysiloxane are present in a content ranging from 10% to 50% by weight, preferably ranging from 20% to 40% by weight, and preferentially ranging from 25% to 40% by weight, relative to the total weight of the composition.

**6.** Composition according to any one of the preceding claims, **characterized in that** the fibres are chosen from silk fibre, cotton fibre, wool fibre, flax fibre, cellulose fibre, polyamide fibre, viscose fibre, acetate fibre, in particular rayon acetate fibre, poly(p-phenyleneterephthalamide) fibre, acrylic fibre, in particular polymethyl methacrylate fibre or poly(2-hydroxyethyl methacrylate) fibre, polyolefin fibre and in particular polyethylene or polypropylene fibre, silica fibre, carbon fibre, in particular in graphite form, polytetrafluoroethylene fibre, insoluble collagen fibre, polyester fibre, polyvinyl chloride fibre or polyvinylidene chloride fibre, polyvinyl alcohol fibre, polyacrylonitrile fibre, chitosan fibre, polyurethane fibre, polyethylene phthalate fibre, fibres formed from a mixture of polymers, and substantially rectilinear rigid fibres, and mixtures thereof.

**7.** Composition according to any one of the preceding claims, **characterized in that** the fibres are fibres of synthetic origin.

**8.** Composition according to any one of the preceding claims, **characterized in that** the fibres have a length ranging from 1 μm to 10 mm, preferably from 0.1 mm to 5 mm and better still from 0.3 to 1 mm.

**9.** Composition according to any one of the preceding claims, **characterized in that** the fibres have a cross section that is within a circle of diameter ranging from 2 nm to 500 μm and preferably ranging from 100 nm to 100 μm.

**10.** Composition according to any one of the preceding claims, **characterized in that** the fibres have a length L and a diameter D such that L/D is chosen in the range from 1.5 to 2500, preferably from 3.5 to 500 and better still from 5 to 150.

**11.** Composition according to any one of the preceding claims, **characterized in that** the fibres are present in a content ranging from 0.5% to 20% by weight, preferably from 0.5% to 15% by weight, preferentially ranging from 0.5% to 8% by weight and more preferentially ranging from 2% to 8% by weight, relative to the total weight of the composition.

**12.** Composition according to any one of the preceding claims, **characterized in that** the fibres and the particles of elastomeric crosslinked organopolysiloxane are present in amounts such that the fibres/particles of crosslinked organopolysiloxane weight ratio ranges from 0.01 to 2, preferably from 0.03 to 1, preferentially from 0.05 to 0.5, and more preferentially from 0.05 to 0.2.

**13.** Composition according to any one of the preceding claims, **characterized in that** the additional powder is chosen from pulverulent dyestuffs, fillers, and mixtures thereof.

**14.** Composition according to any one of the preceding claims, **characterized in that** the additional powder comprises a pulverulent dyestuff.

**15.** Composition according to Claim 13 or 14, **characterized in that** the pulverulent dyestuff is chosen from pigments and nacres, and mixtures thereof.

**16.** Composition according to any one of Claims 13 to 15, **characterized in that** the pulverulent dyestuff is chosen from titanium dioxide, zirconium oxides, cerium oxides, zinc oxides, iron oxides, chromium oxides, manganese violet, ultramarine blue, chromium hydrate, ferric blue, aluminium powder and copper powder.

**17.** Composition according to any one of Claims 13 to 16, **characterized in that** the pulverulent dyestuff is present in a content ranging from 0.5% to 60% by weight, preferably ranging from 0.5% to 50% by weight, preferentially ranging from 0.5% to 40% by weight, more preferentially ranging from 1% to 30% by weight, and even more preferentially ranging from 3% to 25% by weight, relative to the total weight of the composition.

**18.** Composition according to any one of the preceding claims, **characterized in that** the additional powder comprises a filler.

**19.** Composition according to the preceding claim, **characterized in that** the filler is chosen from talc, mica, silica, kaolin, polyamide powders, poly-β-alanine powders, polyethylene powders, tetrafluoroethylene polymer powders, lauroyl-lysine, starch, boron nitride, hollow microspheres of polyvinylidene chloride/acrylonitrile, hollow microspheres of acrylic acid copolymers, ethylene glycol dimethacrylate and lauryl methacrylate copolymer powders, silicone resin microbeads, precipitated calcium carbonate, magnesium carbonate, magnesium hydrocarbonate, hydroxyapatite, hollow silica microspheres, glass or ceramic microcapsules, and metal soaps derived from organic carboxylic acids containing from 8 to 22 carbon atoms, and mixtures thereof.

**20.** Composition according to Claim 18 or 19, **characterized in that** the filler is chosen from mica, polyamide powders, ethylene glycol dimethacrylate and lauryl methacrylate copolymer powders and boron nitride, and mixtures thereof.

**21.** Composition according to any one of Claims 18 to 20, **characterized in that** the filler is present in a content ranging from 0.5% to 40% by weight, preferably ranging from 1% to 35% by weight, and preferentially ranging from 5% to 30% by weight, relative to the total weight of the composition.

**22.** Composition according to any one of the preceding claims, **characterized in that** it comprises a total content of additional powder ranging from 10% to

70% by weight, preferentially ranging from 10% to 60% by weight and more preferentially ranging from 20% to 50% by weight, relative to the total weight of the composition.

**23.** Composition according to any one of the preceding claims, **characterized in that** the particles of elastomeric crosslinked organopolysiloxane and the additional powder are present in contents such that the particles of crosslinked organopolysiloxane/additional powder weight ratio ranges from 0.4/1 to 2/1, preferentially from 0.4/1 to 1.5/1, more preferentially from 0.4/1 to 1.3/1, and even more preferentially from 0.6/1 to 1.3/1.

**24.** Composition according to any one of the preceding claims, **characterized in that** the water is present in a content ranging from 5% to 30% by weight, preferably ranging from 10% to 25% by weight, preferentially ranging from 15% to 25% by weight and more preferentially ranging from 20% to 25% by weight, relative to the total weight of the composition.

**25.** Composition according to any one of the preceding claims, **characterized in that** it comprises a polyol especially containing from 2 to 20 carbon atoms, preferably containing from 2 to 10 carbon atoms and preferentially containing from 2 to 6 carbon atoms.

**26.** Composition according to the preceding claim, **characterized in that** the polyol is chosen from glycerol, propylene glycol, butylene glycol, pentylene glycol, hexylene glycol, dipropylene glycol and diethylene glycol, and mixtures thereof.

**27.** Composition according to Claim 25 or 26, **characterized in that** the polyol is present in a content ranging from 1% to 20% by weight and preferably ranging from 3% to 15% by weight, relative to the total weight of the composition.

**28.** Composition according to any one of the preceding claims, **characterized in that** it comprises a cosmetic ingredient chosen from antioxidants, fragrances, preserving agents, neutralizers, surfactants, waxes, oils, sunscreens, vitamins, moisturizers, self-tanning compounds and anti-wrinkle active agents.

**29.** Composition according to any one of the preceding claims, **characterized in that** the composition has a hardness ranging from 0.07 N to 0.4 N and preferably ranging from 0.1 to 0.35 N, and an elasticity EL ranging from 15% to 80% and preferably ranging from 30% to 70%.

**30.** Composition according to any one of the preceding claims, **characterized in that** the composition is a cosmetic composition.

**31.** Composition according to any one of the preceding claims, **characterized in that** the composition is a makeup or care composition for keratin materials, in particular the skin.

**32.** Composition according to any one of the preceding claims, **characterized in that** the composition is a complexion makeup product (especially a foundation), an eyeshadow, a makeup rouge, a concealer product, a body makeup product, a lip makeup product, a nail makeup product or a hair makeup product.

**33.** Composition according to any one of the preceding claims, **characterized in that** the composition is a skin makeup composition, in particular a complexion makeup composition, especially a foundation.

**34.** Composition according to any one of Claims 1 to 31, **characterized in that** the skincare composition is a skincare product, a skin matting product, an antisun product for the skin, a self-tanning composition or a deodorant product.

**35.** Process for making up keratin materials, especially the skin, comprising the application to the keratin materials, especially to the skin, of a composition according to any one of the preceding claims.

**Patentansprüche**

**1.** Feste Zusammensetzung, die Wasser, feste Partikel eines elastomeren Organopolysiloxans, Fasern und ein ergänzendes Pulver enthält.

**2.** Zusammensetzung nach dem vorhergehenden Anspruch, **dadurch gekennzeichnet, dass** das vernetzte Organopolysiloxan unter den Verbindungen ausgewählt ist, die erhalten werden durch:

- vernetzende Additionsreaktion eines Diorganosiloxans, das mindestens ein an ein Siliciumatom gebundenes Wasserstoffatom enthält, und eines Diorganopolysiloxans, das an ein Siliciumatom gebundene Gruppierungen mit ethylenisch ungesättigter Bindung aufweist;
- vernetzende Kondensationsreaktion unter Wasserstoffabspaltung zwischen einem Diorganopolysiloxan mit endständigen Hydroxygruppen und einem Diorganopolysiloxan, das mindestens ein an ein Siliciumatom gebundenes Wasserstoffatom aufweist;
- vernetzende Kondensationsreaktion eines Diorganopolysiloxans mit endständigen Hydroxygruppen und eines hydrolysierbaren Organopolysiloxans;

- thermische Vernetzung eines Organopolysiloxans;
- Vernetzung eines Organopolysiloxans durch hochenergetische Strahlung.

**3.** Zusammensetzung nach Anspruch 1 oder 2, **dadurch gekennzeichnet, dass** das vernetzte Organopolysiloxan durch vernetzende Additionsreaktion (A) eines Diorganopolysiloxans, das mindestens zwei jeweils an ein Siliciumatom gebundene Wasserstoffatome enthält, und (B) eines Diorganopolysiloxans, das mindestens zwei an ein Siliciumatom gebundene Gruppierungen mit ethylenisch ungesättigter Bindung aufweist, insbesondere in Gegenwart (C) eines Platinkatalysators erhalten wird.

**4.** Zusammensetzung nach einem Ansprüche 1 bis 3, **dadurch gekennzeichnet, dass** das vernetzte Organopolysiloxan durch Umsetzung eines Dimethylpolysiloxans mit endständigen Dimethylvinylsiloxygruppen und eines Methylhydrogenopolysiloxans mit endständigen Trimethylsiloxygruppen in Gegenwart eines Platinkatalysators gebildet wird.

**5.** Zusammensetzung nach einem der vorhergehenden Ansprüche, **dadurch gekennzeichnet, dass** die Partikel des vernetzten elastomeren Organopolysiloxans in einem Mengenanteil von 10 bis 50 Gew.-%, bezogen auf das Gesamtgewicht der Zusammensetzung, vorzugsweise 20 bis 40 Gew.-% und noch bevorzugter 25 bis 40 Gew.-% enthalten sind.

**6.** Zusammensetzung nach einem der vorhergehenden Ansprüche, **dadurch gekennzeichnet, dass** die Fasern unter den Seidenfasern, Baumwollfasern, Wollfasern, Leinenfasern, Cellulosefasern, Polyamidfasern, Viskosefasern, Acetatfasern und insbesondere Fasern aus Rayonacetat, Poly-(*p*-phenylenterephthalamid)-Fasern, Acrylfasern und insbesondere Fasern aus Polymethylmethacrylat oder Poly-2-hydroxyethylmethacrylat, Polyolefinfasern und insbesondere Fasern aus Polyethylen oder Polypropylen, Siliciumdioxidfasern, Kohlenstofffasern, insbesondere Fasern aus in Form von Graphit vorliegendem Kohlenstoff, Polytetrafluorethylenfasern, Fasern aus unlöslichem Collagen, Polyesterfasern, Polyvinylchloridfasern, Polyvinylidenchloridfasern, Polyvinylalkoholfasern, Polyacrylnitrilfasern, Chitosanfasern, Polyurethanfasern, Polyethylenphthalatfasern und Fasern aus Polymerengemischen, im Wesentlichen geraden starren Fasern und deren Gemischen ausgewählt sind.

**7.** Zusammensetzung nach einem der vorhergehenden Ansprüche, **dadurch gekennzeichnet, dass** die Fasern Fasern synthetischer Herkunft sind.

**8.** Zusammensetzung nach einem der vorhergehenden Ansprüche, **dadurch gekennzeichnet, dass** die Fasern eine Länge von 1 μm bis 10 mm, vorzugsweise 0,1 bis 5 mm und besser 0,3 bis 1 mm aufweisen.

**9.** Zusammensetzung nach einem der vorhergehenden Ansprüche, **dadurch gekennzeichnet, dass** der Querschnitt der Fasern in einen Kreis mit einem Durchmesser von 2 nm bis 500 μm und vorzugsweise 100 nm bis 100 μm einbeschrieben werden kann.

**10.** Zusammensetzung nach einem der vorhergehenden Ansprüche, **dadurch gekennzeichnet, dass** die Fasern eine Länge L und einen Durchmesser D aufweisen, die so sind, dass L/D im Bereich von 1,5 bis 2 500, vorzugsweise 3,5 bis 500 und besser 5 bis 150 liegt.

**11.** Zusammensetzung nach einem der vorhergehenden Ansprüche, **dadurch gekennzeichnet, dass** die Fasern in einer Menge von 0,5 bis 20 Gew.-%, bezogen auf das Gesamtgewicht der Zusammensetzung, vorzugsweise 0,5 bis 15 Gew.-%, bevorzugt 0,5 bis 8 Gew.-% und noch bevorzugter 2 bis 8 Gew.-% enthalten sind.

**12.** Zusammensetzung nach einem der vorhergehenden Ansprüche, **dadurch gekennzeichnet, dass** die Fasern und die Partikel eines vernetzten elastomeren Organopolysiloxans in solchen Mengenanteilen enthalten sind, dass das Gewichtsverhältnis Fasern/Partikel eines vernetzten Organopolysiloxans im Bereich von 0,01 bis 2, vorzugsweise 0,03 bis 1, bevorzugt 0,05 bis 0,5 und noch bevorzugter 0,05 bis 0,2 liegt.

**13.** Zusammensetzung nach einem der vorhergehenden Ansprüche, **dadurch gekennzeichnet, dass** das ergänzende Pulver unter den pulverförmigen Farbmitteln, Füllstoffen und deren Gemischen ausgewählt ist.

**14.** Zusammensetzung nach einem der vorhergehenden Ansprüche, **dadurch gekennzeichnet, dass** das ergänzende Pulver ein pulverförmiges Farbmittel umfasst.

**15.** Zusammensetzung nach Anspruch 13 oder 14, **dadurch gekennzeichnet, dass** das pulverförmige Farbmittel unter den Pigmenten, Perlglanzpigmenten und deren Gemischen ausgewählt ist.

**16.** Zusammensetzung nach einem der Ansprüche 13 bis 15, **dadurch gekennzeichnet, dass** das pulverförmige Farbmittel unter Titandioxid, den Oxiden von Zirconium, den Oxiden von Cer, den Oxiden von Zink, den Oxiden von Eisen, den Oxiden von Chrom,

Manganviolett, Ultramarinblau, Chromhydrat, Eisenblau, Aluminiumpulver und Kupferpulver ausgewählt ist.

**17.** Zusammensetzung nach einem der Ansprüche 13 bis 16, **dadurch gekennzeichnet, dass** das pulverförmige Farbmittel in einem Mengenanteil von 0,5 bis 60 Gew.-%, bezogen auf das Gesamtgewicht der Zusammensetzung, vorzugsweise 0,5 bis 50 Gew.-%, noch bevorzugter 0,5 bis 40 Gew.-%, besonders bevorzugt 1 bis 30 Gew.-% und noch bevorzugter 3 bis 25 Gew.-% vorliegt.

**18.** Zusammensetzung nach einem der vorhergehenden Ansprüche, **dadurch gekennzeichnet, dass** das ergänzende Pulver einen Füllstoff umfasst.

**19.** Zusammensetzung nach dem vorhergehenden Anspruch, **dadurch gekennzeichnet, dass** der Füllstoff unter Talk, Glimmer, Kieselsäure, Kaolin, Polyamidpulvern, Poly-β-alaninpulvern, Polyethylenpulvern, Pulver von Tetrafluorethylenpolymeren, Lauroyllysin, Stärke, Bornitrid, Mikrohohlkugeln von Polyvinylidenchlorid/Acrylnitril, Mikrohohlkugeln von Acrylsäure-Copolymeren, Pulvern des Ethylenglycoldimethacrylat/ Laurylmethacrylat- Copolymers, Siliconharz-Mikrokugeln, gefälltem Calciumcarbonat, Magnesiumcarbonat, Magnesiumhydrogencarbonat, Hydroxyapatit, Siliciumdioxid-Mikrohohlkugeln, Glasmikrokapseln oder Keramikmikrokapseln und Metallseifen, die von organischen Carbonsäuren mit 8 bis 22 Kohlenstoffatomen abgeleitet sind, und deren Gemischen ausgewählt ist.

**20.** Zusammensetzung nach Anspruch 18 oder 19, **dadurch gekennzeichnet, dass** der Füllstoff unter Glimmer, Polyamidpulvern, Pulvern des Ethylenglycoldimethacrylat/ Laurylmethacrylat- Copolymers, Bornitrid und deren Gemischen ausgewählt ist.

**21.** Zusammensetzung nach einem der Ansprüche 18 bis 20, **dadurch gekennzeichnet, dass** der Füllstoff in einem Mengenanteil von 0,5 bis 40 Gew.-%, bezogen auf das Gesamtgewicht der Zusammensetzung, vorzugsweise im Bereich von 1 bis 35 Gew.-% und noch bevorzugter im Bereich von 5 bis 30 Gew.-% enthalten ist.

**22.** Zusammensetzung nach einem der vorhergehenden Ansprüche, **dadurch gekennzeichnet, dass** sie eine Gesamtmenge des ergänzenden Pulvers im Bereich von 10 bis 70 Gew.-%, bezogen auf das Gesamtgewicht der Zusammensetzung, vorzugsweise 10 bis 60 Gew.-% und noch bevorzugter 20 bis 50 Gew.-% enthält.

**23.** Zusammensetzung nach einem der vorhergehenden Ansprüche, **dadurch gekennzeichnet, dass** die Partikel eines vernetzten elastomeren Organopolysiloxans und das ergänzende Pulver in solchen Mengenanteilen vorliegen, dass das Gewichtsverhältnis der Partikel eines vernetzten elastomeren Organopolysiloxans und des ergänzenden Pulvers im Bereich von 0,4/ 1 bis 2/ 1, vorzugsweise 0,4/1 bis 1,5/1, noch bevorzugter 0,4/1 bis 1,3/1 und besonders bevorzugt 0,6/1 bis 1,3/ 1 liegt.

**24.** Zusammensetzung nach einem der vorhergehenden Ansprüche, **dadurch gekennzeichnet, dass** das Wasser in einem Mengenanteil von 5 bis 30 Gew.-%, bezogen auf das Gesamtgewicht der Zusammensetzung, vorzugsweise 10 bis 25 Gew.-%, bevorzugt 15 bis 25 Gew.-% und noch bevorzugter 20 bis 25 Gew.-% enthalten ist.

**25.** Zusammensetzung nach einem der vorhergehenden Ansprüche, **dadurch gekennzeichnet, dass** sie ein Polyol enthält, das insbesondere 2 bis 20 Kohlenstoffatome, vorzugsweise 2 bis 10 Kohlenstoffatome und bevorzugt 2 bis 6 Kohlenstoffatome aufweist.

**26.** Zusammensetzung nach dem vorhergehenden Anspruch, **dadurch gekennzeichnet, dass** das Polyol unter Glycerin, Propylenglycol, Butylenglycol, Pentylenglycol, Hexylenglycol, Dipropylenglycol, Diethylenglycol und deren Gemischen ausgewählt ist.

**27.** Zusammensetzung nach Anspruch 25 oder 26, **dadurch gekennzeichnet, dass** das Polyol in einem Mengenanteil von 1 bis 20 Gew.-%. bezogen auf das Gesamtgewicht der Zusammensetzung, und vorzugsweise 3 bis 15 Gew.-% enthalten ist.

**28.** Zusammensetzung nach einem der vorhergehenden Ansprüche, **dadurch gekennzeichnet, dass** sie eine kosmetischen Bestandteil enthält, der unter den Antioxidantien, Parfums, Konservierungsmitteln, Neutralisationsmitteln, grenzflächenaktiven Stoffen, Wachsen, Ölen, Sonnenschutzmitteln, Vitaminen, Hydratisierungsmitteln, Selbstbräunungsmitteln und Wirkstoffen gegen Falten ausgewählt ist.

**29.** Zusammensetzung nach einem der vorhergehenden Ansprüche, **dadurch gekennzeichnet, dass** die Zusammensetzung eine Härte von 0,07 bis 0,4 N und vorzugsweise 0,1 bis 0,35 N und eine Elastizität EL von 15 bis 80 % und vorzugsweise 30 bis 70 % aufweist,

**30.** Zusammensetzung nach einem der vorhergehenden Ansprüche, **dadurch gekennzeichnet, dass** die Zusammensetzung eine kosmetische Zusammensetzung ist.

**31.** Zusammensetzung nach einem der vorhergehenden Ansprüche, **dadurch gekennzeichnet, dass** die Zusammensetzung eine Zusammensetzung zum Schminken oder für die Pflege von Keratinsubstanzen und insbesondere der Haut ist.

**32.** Zusammensetzung nach einem der vorhergehenden Ansprüche, **dadurch gekennzeichnet, dass** die Zusammensetzung ein Schminkprodukt für den Teint (insbesondere Make-up), ein Lidschatten, ein Wangenrouge, ein Produkt gegen Augenringe, ein Produkt zum Schminken des Körpers, ein Produkt zum Schminken der Lippen, ein Produkt zum Schminken der Nägel, ein Produkt zum Schminken der Nägel, ein Produkt zum Schminken der Haare ist.

**33.** Zusammensetzung nach einem der vorhergehenden Ansprüche, **dadurch gekennzeichnet, dass** es sich bei der Zusammensetzung um eine Zusammensetzung zum Schminken der Haut und insbesondere ein Schminkprodukt für den Teint, besonders ein Make-up handelt.

**34.** Zusammensetzung nach einem der vorhergehenden Ansprüche 1 bis 31, **dadurch gekennzeichnet, dass** es sich bei der Zusammensetzung für die Pflege der Haut um ein Pflegeprodukt für die Haut, ein mattierendes Produkt für die Haut, ein Sonnenschutzprodukt für die Haut, eine Selbstbräunungszusammensetzung oder ein desodorierendes Produkt handelt.

**35.** Verfahren zum Schminken von Keratinsubstanzen, insbesondere der Haut, das das Auftragen einer Zusammensetzung nach einem der vorhergehenden Zusammensetzung auf die Keratinsubstanzen und insbesondere die Haut umfasst.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 02053126 A
- EP 1064930 A
- EP 295886 A
- JP 61194009 A
- EP 242219 A
- EP 381166 A
- JP 10175816 A
- US 5928660 A
- EP 6921217 A
- EP 686858 A
- US 5472798 A
- US 3802841 A
- FR 2079785 A
- EP 0360728 A1
- EP 0549494 A


### Littérature non-brevet citée dans la description


- **R. PIGEON ; P. ALLARD.** *Chimie Macromoléculaire Appliquée,* 1974, vol. 40/41 (600), 139-158